# EUROPEAN PATENT SPECIFICATION

(11) **EP 2 844 693 B1**
(45) Date of publication and mention of the grant of the patent: **30.09.2015**
(21) Application number: 12735375.3
(22) Date of filing: 26.06.2012
(51) Int. Cl.: C08K 5/18, A61F 2/16, C08L 33/06, C08L 83/04

(54) **2-AMINO BENZOPHENONE UV-ABSORBERS FOR OPHTHALMIC LENS MATERIALS**
2-AMINOBENZOPHENON-UV-ABSORBER FÜR BRILLENGLASMATERIALIEN
AGENTS 2-AMINO BENZOPHÉNONE ABSORBANT LES UV POUR MATÉRIAUX DE LENTILLES OPHTALMIQUES

(43) Date of publication of application: 11.03.2015
(73) Proprietor: Novartis AG, 4056 Basel (CH)
(72) Inventor: FREEMAN, Charles, Granbury, Texas 76049 (US)
(74) Representative: Hanna, Peter William Derek
(86) International application number: PCT/US2012/044132
(87) International publication number: WO 2014/003714

(56) References cited:
- US-A- 5 470 932
- US-B2- 7 037 954

## Description

### Field of the Invention

This invention is directed to ophthalmic lens materials. In particular, this invention relates to ultraviolet light absorbers that are suitable for use in ophthalmic lens materials.

### Background of the Invention

Many UV light absorbers are known as ingredients for polymeric materials used to make ophthalmic lenses and, in particular, intraocular lenses. UV absorbers are preferably covalently bound to the polymeric network of the lens material instead of simply physically entrapped in the material to prevent the absorber from migrating, phase separating or leaching out of the lens material. Such stability is particularly important for implantable ophthalmic lenses where the leaching of the UV absorber may present both toxicological issues and lead to the loss of UV blocking activity in the implant.

Numerous copolymerizable benzotriazole, benzophenone and triazine UV absorbers are known. Many of these UV absorbers contain conventional olefinic polymerizable groups, such as methacrylate, acrylate, methacrylamide, acrylamide or styrene groups. Copolymerization with other ingredients in the lens materials, typically with a radical initiator, incorporates the UV absorbers into the resulting polymer chain. Incorporation of additional functional groups on a UV absorber may influence one or more of the UV absorber's UV absorbing properties, solubility or reactivity. If the UV absorber does not have sufficient solubility in the remainder of the ophthalmic lens material ingredients or polymeric lens material, the UV absorber may coalesce into domains that could interact with light and result in decreased optical clarity of the lens.

Examples of polymeric ophthalmic lens materials that incorporate UV absorbers can be found in U.S. Patent Nos. 5,290,892; 5,331,073 and 5,693,095.

In addition to blocking UV light, some ophthalmic lenses also block blue light. See, for example, U.S. Patent Nos. 5,470,932 and 5,543,504. These lenses block both types of light by using two chromophores: a UV absorber and a yellow dye.

There is a need for UV absorbers that are suitable for use in implantable ophthalmic lenses and are capable of blocking not only UV light (400 nm and below) but also blocking at least some light between 400 - 450 nm.

### Summary of the Invention

The present invention provides ophthalmic device materials comprising UV absorbers that block not only UV light but also at least some light in the 400 - 450 nm range. The ophthalmic device materials are suitable for a variety of ophthalmic devices, including contact lenses, and are particularly useful in implantable lenses, such as intraocular lenses (IOLs).

An ophthalmic device material comprising
a) 0.1 to 5% (w/w) of a UV absorber of formula (I): 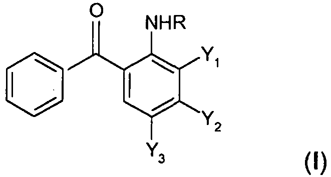 wherein in formula (I):
   R is H, C₁-C₄ alkyl, -C(=O)C(=CH₂)X, or
      -C(=O)O-(CH₂)ₙO-C(=O)C(=CH₂)X;
   n is 1 - 6;
   X is H, CH₃, or CH₂CH₃;
   Y₁, Y₂ independently are H, Br, Cl, F, I, OCH₃, NO₂, -C(=O)C(=CH₂)X, or -C(=O)O-(CH₂)ₙO-C(=O)C(=CH₂)X; and
   Y₃ is H, Br, Cl, F, I, OCH₃, or NO₂;
   provided that no more than one of R, Y₁, and Y₂ may be -C(=O)C(=CH₂)X or-C(=O)O-(CH₂)ₙO-C(=O)C(=CH₂)X; and
b) a device-forming monomer selected from the group consisting of acrylic monomers and silicone-containing monomers.

### Brief Description of the Drawings

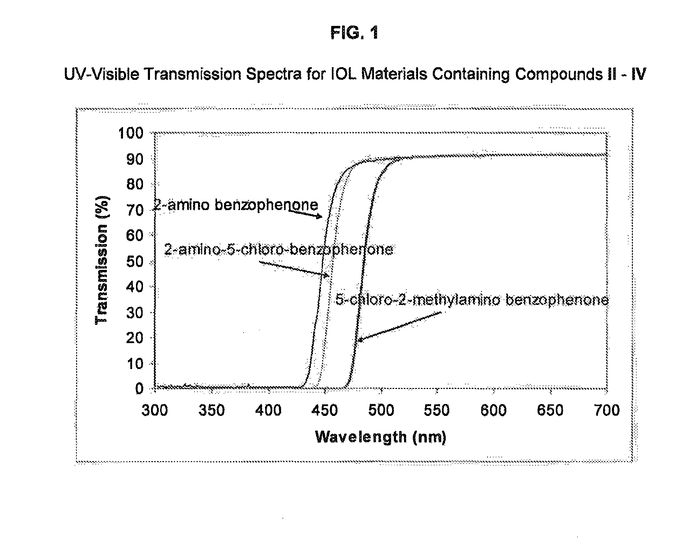

Fig. 1 shows the UV/VIS spectra of three different 2-amino benzophenone UV absorbers in an acrylic IOL material.

### Detailed Description of the Invention

Unless indicated otherwise, all ingredient amounts expressed in percentage terms are presented as % w/w.

The UV absorbers contained in the ophthalmic device materials of the present invention have the structure shown in formula I. 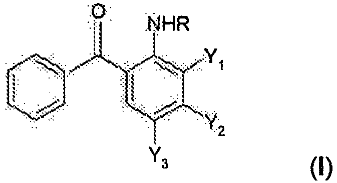 wherein:
R is H, C₁-C₄ alkyl, -C(=O)C(=CH₂)X, or
   -C(=O)O-(CH₂)ₙO-C(=O)C(=CH₂)X;
n is 1 - 6;
X is H, CH₃, or CH₂CH₃;
Y₁, Y₂ independently are H, Br, Cl, F, I, OCH₃, NO₂, -C(=O)C(=CH₂)X, or
   -C(=O)O-(CH₂)ₙO-C(=O)C(=CH₂)X; and
Y₃ is H, Br, Cl, F, I, OCH₃, or NO₂;
provided that no more than one of R, Y₁, and Y₂ may be -C(=O)C(=CH₂)X or -C(=O)O-(CH₂)ₙO-C(=O)C(=CH₂)X.

Preferably,
R is H or C₁-C₄ alkyl;
n is 1 - 6;
X is H, CH₃, or CH₂CH₃;
Y₁, Y₂ independently are H, Br, Cl, F, I, OCH₃, NO₂, -C(=O)C(=CH₂)X, or -C(=O)O-(CH₂)ₙO-C(=O)C(=CH₂)X; and
Y₃ is Br, Cl, F, I, OCH₃, or NO₂;
provided that no more than one of Y₁ and Y₂ may be -C(=O)C(=CH₂)X or -C(=O)O-(CH₂)ₙO-C(=O)C(=CH₂)X.

More preferably,
R is H or C₁-C₄ alkyl;
n is 1 -6;
X is H or CH₃;
Y₁, Y₂ independently are H, Br, Cl, F, I, OCH₃, NO₂, -C(=O)C(=CH₂)X, or -C(=O)O-(CH₂)ₙO-C(=O)C(=CH₂)X; and
Y₃ is Cl;
provided that no more than one of Y₁ and Y₂ may be -C(=O)C(=CH₂)X or -C(=O)O-(CH₂)ₙO-C(=O)C(=CH₂)X.

Representative compounds of formula I are the following:

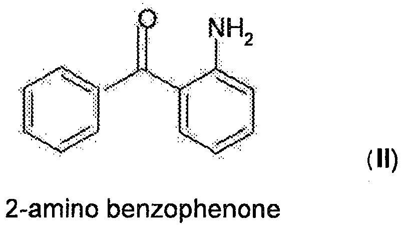

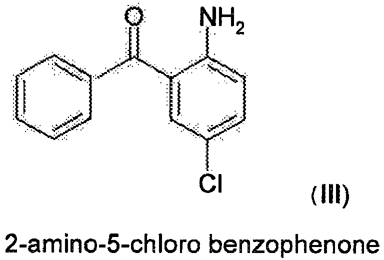

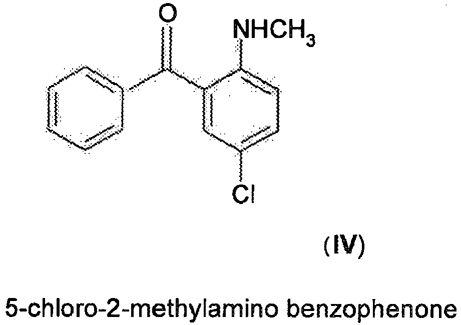

Compounds of formula (I) are commercially available or can be prepared using methods known in the art. For example, a synthetic pathway to prepare the polymerizable compounds of the present invention is to place the desired amino benzophenone into a flask in dichloromethane. Add a slight excess of amine catalyst followed by acryloyl chloride. Alternatively, the polymerizable compounds can be prepared by adding isocyanatoethyl methacrylate to the desired amino benzophenone.

The ophthalmic device materials of the present invention are particularly suitable for use in IOLs. IOL materials will generally contain from 0.1 to 5 % (w/w) of a UV absorber of formula I. Preferably, IOL materials will contain from 0.1 to 4 % (w/w) of a UV absorber of the present invention. Most preferably, IOL materials will contain from 1 to 3 % (w/w) of a UV absorber of the present invention.

Ophthalmic device materials are prepared by copolymerizing a UV absorber of formula (I) with a device-forming monomer, a cross-linking agent, and, if desired, a blue-light blocking chromophore.

Many device-forming monomers are known in the art and include both acrylic and silicone-containing monomers among others. See, for example, U.S. Nos. 7,101,949; 7,067,602; 7,037,954; 6,872,793 6,852,793; 6,846,897; 6,806,337; 6,528,602; and 5,693,095. In the case of IOLs, any known IOL device material is suitable for use in the compositions of the present invention. Preferably, the ophthalmic device materials comprise an acrylic or methacrylic device-forming monomer. More preferably, the device-forming monomers comprise a monomer of formula V: 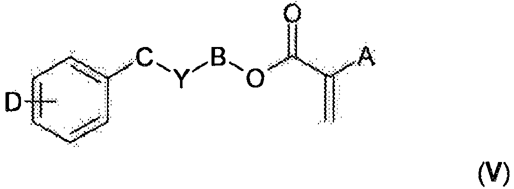 wherein in formula V:
A is H, CH₃, CH₂CH₃, or CH₂OH;
B is (CH₂)ₘ or [O(CH₂)₂]_{z};
C is (CH₂)_{w};
m is 2 - 6;
z is 1 - 10;
Y is nothing, O, S, or NR', provided that if Y is O, S, or NR', then B is (CH₂)ₘ;
R' is H, CH₃, C_{n'}H_{2n'+1} (n'=1-10), iso-OC₃H₇, C₆H₅, or CH₂C₆H₅;
w is 0 - 6, provided that m + w ≤8; and
D is H, C₁ - C₄ alkyl, C₁ - C₄ alkoxy, C₆H₅, CH₂C₆H₅ or halogen.

Preferred monomers of formula V are those wherein A is H or CH₃, B is (CH₂)ₘ, m is 2 - 5, Y is nothing or O, w is 0 - 1, and D is H. Most preferred are 2-phenylethyl methacrylate; 4-phenylbutyl methacrylate; 5-phenylpentyl methacrylate; 2-benzyloxyethyl methacrylate; and 3-benzyloxypropyl methacrylate; and their corresponding acrylates.

Monomers of formula V are known and can be made by known methods. For example, the conjugate alcohol of the desired monomer can be combined in a reaction vessel with methyl methacrylate, tetrabutyl titanate (catalyst), and a polymerization inhibitor such as 4-benzyloxy phenol. The vessel can then be heated to facilitate the reaction and distill off the reaction by-products to drive the reaction to completion. Alternative synthesis schemes involve adding methacrylic acid to the conjugate alcohol and catalyzing with a carbodiimide or mixing the conjugate alcohol with methacryloyl chloride and a base such as pyridine or triethylamine.

The ophthalmic device materials of the present invention generally comprise a total of at least about 75%, preferably at least about 80%, of device-forming monomers:

In addition to a UV absorber of formula I and a device-forming monomer, the device materials of the present invention generally comprise a cross-linking agent. The cross-linking agent used in the device materials of this invention may be any terminally ethylenically unsaturated compound having more than one unsaturated group. Suitable cross-linking agents include, for example: ethylene glycol dimethacrylate; diethylene glycol dimethacrylate; allyl methacrylate; 1,3-propanediol dimethacrylate; 2,3-propanediol dimethacrylate; 1,6-hexanediol dimethacrylate; 1,4-butanediol dimethacrylate; CH₂=C(CH₃)C(=O)O-(CH₂CH₂O)ₚ-C(=O)C(CH₃)=CH₂ where p = 1 - 50; and CH₂=C(CH₃)C(=O)O(CH₂)ₜO-C(=O)C(CH₃)=CH₂ where t = 3 - 20; and their corresponding acrylates. A preferred cross-linking monomer is CH₂=C(CH₃)C(=O)O-(CH₂CH₂O)ₚ-C(=O)C(CH₃)=CH₂ where p is such that the number-average molecular weight is about 400, about 600, or about 1000.

Generally, the total amount of the cross-linking component is at least 0.1% by weight and, depending on the identity and concentration of the remaining components and the desired physical properties, can range to about 20% by weight. The preferred concentration range for the cross-linking component is 0.1 - 17% (w/w).

Suitable polymerization initiators for the device materials of the present invention include thermal initiators and photoinitiators. Preferred thermal initiators include peroxy free-radical initiators, such as t-butyl (peroxy-2-ethyl)hexanoate and di-(tert-butylcyclohexyl) peroxydicarbonate (commercially available as Perkadox^{®} 16 from Akzo Chemicals Inc., Chicago, Illinois). Initiators are typically present in an amount of about 5% (w/w) or less. Because free-radical initiators do not become chemically a part of the polymers formed, the total amount of initiator is customarily not included when determining the amounts of other ingredients.

The device materials of the present invention optionally contain a reactive colorant. Suitable reactive blue-light absorbing compounds include those described in U.S. Patent No. 5,470,932. Blue-light absorbers are typically present in the device materials of the present invention in an amount from about 0.01 - 0.5 % (weight).

IOLs constructed of the device materials of the present invention can be of any design capable of being rolled or folded into a small cross section that can fit through a relatively smaller incision. For example, the IOLs can be of what is known as a one piece or multipiece design, and comprise optic and haptic components. The optic is that portion which serves as the lens. The haptics are attached to the optic and hold the optic in its proper place in the eye. The optic and haptic(s) can be of the same or different material. A multipiece lens is so called because the optic and the haptic(s) are made separately and then the haptics are attached to the optic. In a single piece lens, the optic and the haptics are formed out of one piece of material. Depending on the material, the haptics are then cut, or lathed, out of the material to produce the IOL.

In addition to IOLs, the device materials of the present invention are also suitable for use in other ophthalmic devices, such as contact lenses, keratoprostheses, and corneal inlays or rings.

The invention will be further illustrated by the following examples, which are intended to be illustrative, but not limiting.

### Examples 1 - 3

### Preparation of IOL Materials Incorporating UV Absorbers of Formulas II - IV

The monomers 2-phenylethyl acrylate (66.1 wt.%), 2-phenylethyl methacrylate (30.6 wt.%), and the crosslinker 1,4-butanediol diacrylate (3.3 wt.%) are weighed into a vial and shaken to mix. Azobisisobutyronitrile thermal initiator (2 wt.%) is then added and shaken to mix. Nitrogen is bubbled into the mix. This master batch is then divided into separate clean vials and approximately 3 wt. % of the desired benzophenone absorber is added. The solutions are then placed into polypropylene molds and then into an oven for curing. The cured slabs are placed into methanol for 25 minutes. Fresh methanol is added then decanted and the slabs allowed to slowly dry. Subsequently, they are placed into a vacuum oven to remove the solvent. The UV-visible transmission spectrum of each lens material was measured. The measurement was performed from 850 to 250 nm in 1-mm quartz cuvettes using a Perkin-Elmer Lambda 35 UV-Visible Spectrophotometer. The results are shown in Figure 1.

## Claims

1. An ophthalmic device material comprising
a) 0.1 to 5% (w/w) of a UV absorber of formula (I): 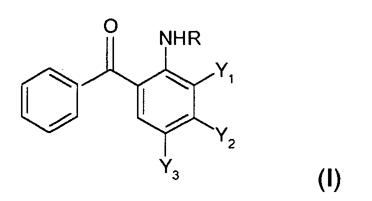 wherein in formula (I):
R is H, C₁-C₄ alkyl, -C(=O)C(=CH₂)X, or -C(=O)O-(CH₂)ₙO-C(=O)C(=CH₂)X;
n is 1 - 6;
X is H, CH₃, or CH₂CH₃;
Y₁, Y₂ independently are H, Br, Cl, F, I, OCH₃, NO₂, -C(=O)C(=CH₂)X, or -C(=O)O-(CH₂)ₙO-C(=O)C(=CH₂)X; and
Y₃ is H, Br, Cl, F, I, OCH₃, or NO₂;
provided that no more than one of R, Y₁, and Y₂ may be -C(=O)C(=CH₂)X or-C(=O)O-(CH₂)ₙO-C(=O)C(=CH₂)X; and
b) a device-forming monomer selected from the group consisting of acrylic monomers and silicone-containing monomers.

2. The ophthalmic device material of Claim 1 wherein in formula (I)
R is H or C₁-C₄ alkyl;
n is 1 - 6;
X is H, CH₃, or CH₂CH₃;
Y₁, Y₂ independently are H, Br, Cl, F, I, OCH₃, NO₂, -C(=O)C(=CH₂)X, or -C(=O)O-(CH₂)ₙO-C(=O)C(=CH₂)X; and
Y₃ is Br, Cl, F, I, OCH₃, or NO₂;
provided that no more than one of Y₁ and Y₂ may be -C(=O)C(=CH₂)X or -C(=O)O-(CH₂)ₙO-C(=O)C(=CH₂)X.

3. The ophthalmic device material of Claim 2 wherein in formula (I)
R is H or C₁-C₄ alkyl;
n is 1 - 6;
X is H or CH₃;
Y₁, Y₂ independently are H, Br, Cl, F, I, OCH₃, NO₂, -C(=O)C(=CH₂)X, or -C(=O)O-(CH₂)ₙO-C(=O)C(=CH₂)X; and
Y₃ is Cl;
provided that no more than one of Y₁ and Y₂ may be -C(=O)C(=CH₂)X or -C(=O)O-(CH₂)ₙO-C(=O)C(=CH₂)X.

4. The ophthalmic device material of Claim 1 wherein the UV absorber is selected from the group consisting of: 2-amino benzophenone; 2-amino-5-chloro benzophenone; and 2-methylamino-5-chloro benzophenone.

5. The ophthalmic device material of Claim 1 wherein the ophthalmic device material comprises from 0.1 to 4 % (w/w) of the UV absorber.

6. The ophthalmic device material of Claim 5 wherein the ophthalmic device material comprises from 1 to 3 % (w/w) of the UV absorber.

7. The ophthalmic device material of Claim 1 wherein the ophthalmic device material comprises a device-forming monomer of formula [**V**]**:** 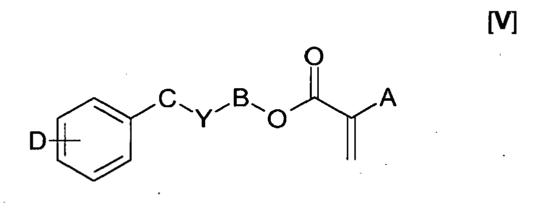 where in formula [**V**]**:**
A is H, CH₃, CH₂CH₃, or CH₂OH;
B is (CH₂)ₘ or [O(CH₂)₂]_{z};
C is (CH₂)_{w};
m is 2 - 6;
z is 1 - 10;
Y is nothing, O, S, or NR', provided that if Y is O, S, or NR', then B is (CH₂)ₘ;
R' is H, CH₃, C_{n'}H_{2n'+1} (n'=1-10), iso-OC₃H₇, C₆H₅, or CH₂C₆H₅;
w is 0 - 6, provided that m + w ≤8; and
D is H, C₁ - C₄ alkyl, C₁ - C₄ alkoxy, C₆H₅, CH₂C₆H₅ or halogen.

8. The ophthalmic device material of Claim 7 wherein in formula [**V**]:
A is H or CH₃;
B is (CH₂)ₘ;
m is 2 - 5;
Y is nothing or O;
w is 0 - 1; and
D is H.

9. The ophthalmic device material of Claim 8 wherein the ophthalmic device material comprises a monomer selected from the group consisting of: 2-phenylethyl methacrylate; 4-phenylbutyl methacrylate; 5-phenylpentyl methacrylate; 2-benzyloxyethyl methacrylate; 3-benzyloxypropyl methacrylate; 2-phenylethyl acrylate; 4-phenylbutyl acrylate; 5-phenylpentyl acrylate; 2-benzyloxyethyl acrylate; and 3-benzyloxypropyl acrylate.

10. The ophthalmic device material of Claim 1 wherein the ophthalmic device material comprises a cross-linking agent.

11. The ophthalmic device material of Claim 1 wherein the ophthalmic device material comprises a reactive blue-light absorbing compound.

12. An ophthalmic device comprising an ophthalmic device material of Claim 1, wherein the ophthalmic device is selected from the group consisting of an intraocular lens; a contact lens; a keratoprosthesis; and a corneal inlay or ring.

13. The ophthalmic device of Claim 12 wherein the ophthalmic device is an intraocular lens.

## Patentansprüche

1. Material für eine ophthalmische Vorrichtung, umfassend:
a) 0,1 bis 5 % (Gew./Gew.) eines UV-Absorbers mit der Formel (I): 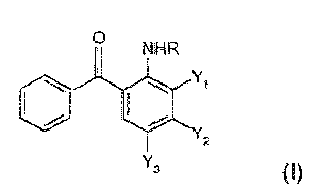 wobei in der Formel (I):
R für H, C₁-C₄ Alkyl, -C(=O)C(=CH₂)X oder -C(=O)O-(CH₂)ₙO-C(=O)C(=CH₂)X steht;
n für 1 bis 6 steht;
X für H, CH₃ oder CH₂CH₃ steht;
Y₁, Y₂ unabhängig voneinander für H, Br, Cl, F, I, OCH₃, NO₂, -C(=O)C(=CH₂)X oder -C(=O)O-(CH₂)ₙO-C(=O)C(=CH₂)X stehen; und
Y₃ für H, Br, Cl, F, I, OCH₃ oder NO₂ steht;
vorausgesetzt, dass höchstens eines aus der Gruppe von R, Y₁ und Y₂ für - C(=O)C(=CH₂)X oder -C(=O)O-(CH₂)ₙO-C(=O)C(=CH₂)X stehen kann; und
b) ein vorrichtungsbildendes Monomer, das aus einer Gruppe, die aus Acrylmonomeren und Silikon enthaltenden Monomeren besteht, ausgewählt wurde.

2. Material für eine ophthalmische Vorrichtung gemäß Anspruch 1, wobei in der Formel (I):
R für H oder C₁-C₄ Alkyl steht;
n für 1 bis 6 steht;
X für H, CH₃ oder CH₂CH₃ steht;
Y₁, Y₂ unabhängig voneinander für H, Br, Cl, F, I, OCH₃, NO₂, -C(=O)C(=CH₂)X oder -C(=O)O-(CH₂)ₙO-C(=O)C(=CH₂)X stehen; und
Y₃ für Br, Cl, F, I, OCH₃ oder NO₂ steht;
vorausgesetzt, dass höchstens eines aus der Gruppe von Y₁ und Y₂ für -C(=O)C(=CH₂)X oder -C(=O)O-(CH₂)ₙO-C(=O)C(=CH₂)X stehen kann.

3. Material für eine ophthalmische Vorrichtung gemäß Anspruch 1, wobei in der Formel (I):
R für H oder C₁-C₄ Alkyl steht;
n für 1 bis 6 steht;
X für H oder CH₃ steht;
Y₁, Y₂ unabhängig voneinander für H, Br, Cl, F, I, OCH₃, NO₂, -C(=O)C(=CH₂)X, oder -C(=O)O-(CH₂)ₙO-C(=O)C(=CH₂)X stehen; und
Y₃ für Cl steht;
vorausgesetzt, dass höchstens eines aus der Gruppe von Y₁ und Y₂ für -C(=O)C(=CH₂)X oder -C(=O)O-(CH₂)ₙO-C(=O)C(=CH₂)X stehen kann.

4. Material für eine ophthalmische Vorrichtung gemäß Anspruch 1, wobei der UV-Absorber aus der Gruppe ausgewählt wird, die aus 2-Aminobenzophenon, 2-Amino-5-Chlorobenzophenon und 2-Methylamino-5-Chlorobenzophenon besteht.

5. Material für eine ophthalmische Vorrichtung gemäß Anspruch 1, wobei das Material für die ophthalmische Vorrichtung von 0,1 bis 4 % (Gew./Gew.) des UV-Absorbers enthält.

6. Material für eine ophthalmische Vorrichtung gemäß Anspruch 5, wobei das Material für die ophthalmische Vorrichtung von 1 bis 3 % (Gew./Gew.) des UV-Absorbers enthält.

7. Material für eine ophthalmische Vorrichtung gemäß Anspruch 1, wobei das Material für die ophthalmische Vorrichtung ein vorrichtungsbildendes Monomer gemäß der Formel [V] umfasst: 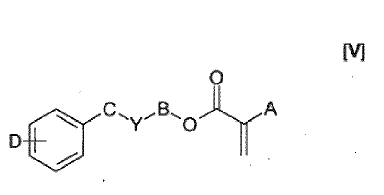 wobei in der Formel [V]:
A für H, CH₃, CH₂CH₃ oder CH₂OH steht;
B für (CH₂)ₘ oder [O(CH₂)₂]_{z} steht;
C für (CH₂)_{w} steht;
m für 2 bis 6 steht;
z für 1 bis 10 steht;
Y steht für Nichts, O, S, oder NR', vorausgesetzt, dass, wenn Y für O, S, oder NR' steht,
dann steht B für (CH₂)ₘ;
R' für H, CH₃, C_{n'}H_{2n'+}1 (n'=1-10), Iso-OC₃H₇, C₆H₅ oder CH₂C₆H₅ steht;
w für 0 bis 6 steht, vorausgesetzt, dass m + w ≤ 8; und
D für H, C₁-C₄ Alkyl, C₁-C₄ Alkoxy, C₆H₅, CH₂C₆H₅ oder Halogen steht.

8. Material für eine ophthalmische Vorrichtung gemäß Anspruch 7, wobei in der Formel [V]:
A für H oder CH₃ steht;
B für (CH₂)ₘ steht;
m für 2 bis 5 steht;
Y für Nichts oder O steht;
w für 0 bis 1 steht; und
D für H steht.

9. Material für eine ophthalmische Vorrichtung gemäß Anspruch 8, wobei das Material für die ophthalmische Vorrichtung aus einer Gruppe ausgewählt wird, die aus 2-Phenylethylmethacrylat, 4-Phenylbutylmethacrylat, 5-Phenylpentylmethacrylat, 2-Benzyloxyethylmethacrylat, 3-Benzyloxypropylmethacrylat, 2-Phenylethylacrylat, 4-Phenylbutylacrylat, 5-Phenylpentylacrylat, 2-Benzyloxyethylacrylat und 3-Benzyloxypropylacrylat besteht.

10. Material für eine ophthalmische Vorrichtung gemäß Anspruch 1, wobei das Material für die ophthalmische Vorrichtung ein Vernetzungsmittel umfasst.

11. Material für eine ophthalmische Vorrichtung gemäß Anspruch 1, wobei das Material für die ophthalmische Vorrichtung eine reaktive, blaues Licht absorbierende Verbindung umfasst.

12. Ophtalmische Vorrichtung, umfassend ein Material für eine ophthalmische Vorrichtung gemäß Anspruch 1, wobei die ophthalmische Vorrichtung aus der Gruppe ausgewählt wird, die aus Intraokularlinse, Kontaktlinse, Keratoprothese und Hornhautinlay oder Hornhautring besteht.

13. Ophtalmische Vorrichtung gemäß Anspruch 12, wobei es sich bei der ophthalmischen Vorrichtung um eine Intraokularlinse handelt.

## Revendications

1. Matériau de dispositif ophtalmique comprenant
a) 0,1 à 5 % (p/p) d'un absorbeur UV de formule (I) : 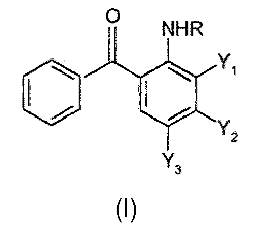 où, dans la formule (I) :
R est H, un alkyle en C₁ à C₄, -C(=O)C(=CH₂)X, ou -C(=O)O-(CH₂)ₙO-C(=O)C(=CH₂)X ;
n vaut de 1 à 6 ;
X est H, CH₃ ou CH₂CH₃ ;
Y₁ et Y₂ sont indépendamment H, Br, Cl, F, I, OCH₃, NO₂, -C(=O)C(=CH₂)X,
ou -C(=O)O-(CH₂)ₙO-C(=O)C(=CH₂)X ; et
Y₃ est H, Br, Cl, F, I, OCH₃, ou NO₂ ;
sous réserve qu'au plus un parmi R, Y₁ et Y₂ puisse être -C(=O)C(=CH₂)X ou -C(=O)O-(CH₂)ₙO-C(=O)C(=CH₂)X ; et
b) un monomère formant un dispositif, choisi dans l'ensemble constitué par les monomères acryliques et les monomères siliconés.

2. Matériau de dispositif ophtalmique selon la revendication 1, dans lequel, dans la formule (I),
R est H ou un alkyle en C₁ à C₄ ;
n vaut de 1 à 6 ;
X est H, CH₃ ou CH₂CH₃ ;
Y₁ et Y₂ sont indépendamment H, Br, Cl, F, I, OCH₃, NO₂, -C(=O)C(=CH₂)X, ou -C(=O)O-(CH₂)ₙO-C(=O)C(=CH₂)X ; et
Y₃ est Br, Cl, F, I, OCH₃, ou NO₂ ;
sous réserve qu'au plus un parmi Y₁ et Y₂ puisse être -C(=O)C(=CH₂)X ou -C(=O)O-(CH₂)ₙO-C(=O)C(=CH₂)X.

3. Matériau de dispositif ophtalmique selon la revendication 2, dans lequel, dans la formule (I),
R est H ou un alkyle en C₁ à C₄ ;
n vaut de 1 à 6 ;
X est H ou CH₃ ;
Y₁ et Y₂ sont indépendamment H, Br, Cl, F, I, OCH₃, NO₂, -C(=O)C(=CH₂)X, ou -C(=O)O-(CH₂)ₙO-C(=O)C(=CH₂)X ; et
Y₃ est Cl;
sous réserve qu'au plus un parmi Y₁ et Y₂ puisse être -C(=O)C(=CH₂)X ou -C(=O)O-(CH₂)ₙO-C(=O)C(=CH₂)X.

4. Matériau de dispositif ophtalmique selon la revendication 1, dans lequel l'absorbeur UV est choisi dans l'ensemble constitué par : la 2-aminobenzophénone ; la 2-amino-5-chlorobenzophénone ; et la 2-méthylamino-5-chlorobenzophénone.

5. Matériau de dispositif ophtalmique selon la revendication 1, lequel matériau de dispositif ophtalmique comprend de 0,1 à 4 % (p/p) de l'absorbeur UV.

6. Matériau de dispositif ophtalmique selon la revendication 5, lequel matériau de dispositif ophtalmique comprend de 1 à 3 % (p/p) de l'absorbeur UV.

7. Matériau de dispositif ophtalmique selon la revendication 5, lequel matériau de dispositif ophtalmique comprend un monomère formant un dispositif de formule [V] : 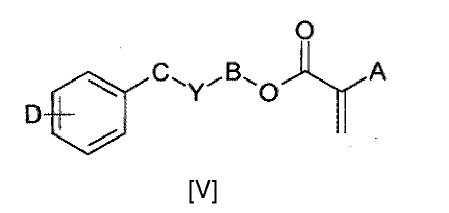 où, dans la formule [V] :
A est H, CH₃, CH₂CH₃ ou CH₂OH ;
B est (CH₂)ₘ ou [O(CH₂)₂]_{z} ;
C est (CH₂)_{w} ;
m vaut de 2 à 6 ;
z vaut de 1 à 10 ;
Y n'existe pas ou est O, S ou NR', sous réserve que, si Y est O, S ou NR', alors B est (CH₂)ₘ ;
R' est H, CH₃, C_{n'}H_{2n'+1} (n' = 1-10), iso-OC₃H₇, C₆H₅, ou CH₂C₆H₅ ;
w vaut de 0 à 6, sous réserve que m + w ≤ 8 ; et
D est H, un alkyle en C₁ à C₄, alcoxy en C₁ à C₄, C₆H₅ ou CH₂C₆H₅ ou un halogène.

8. Matériau de dispositif ophtalmique selon la revendication 7, dans lequel, dans la formule [V] :
A est H ou CH₃ ;
B est (CH₂)ₘ ;
m vaut de 2 à 5 ;
Y n'existe pas ou est O ;
w vaut de 0 à 1 ; et
D est H.

9. Matériau de dispositif ophtalmique selon la revendication 8, lequel matériau de dispositif ophtalmique comprend un monomère choisi dans l'ensemble constitué par : le méthacrylate de 2-phényléthyle ; le méthacrylate de 4-phénylbutyle ; le méthacrylate de 5-phénylpentyle ; le méthacrylate de 2-benzyloxyéthyle ; le méthacrylate de 3-benzyloxypropyle ; l'acrylate de 2-phényléthyle ; l'acrylate de 4-phénylbutyle ; l'acrylate de 5-phénylpentyle ; l'acrylate de 2-benzyloxyéthyle ; et l'acrylate de 3-benzyloxypropyle.

10. Matériau de dispositif ophtalmique selon la revendication 1, lequel matériau de dispositif ophtalmique comprend un agent de réticulation.

11. Matériau de dispositif ophtalmique selon la revendication 1, lequel matériau de dispositif ophtalmique comprend un composé absorbant la lumière bleue.

12. Matériau de dispositif ophtalmique selon la revendication 1, lequel matériau de dispositif ophtalmique est choisi dans l'ensemble constitué par un cristallin artificiel ; une lentille de contact ; une kératoprothèse ; et un implant ou un anneau cornéen.

13. Dispositif ophtalmique selon la revendication 12, lequel dispositif ophtalmique est un cristallin artificiel.
